# EUROPEAN PATENT SPECIFICATION

(11) **EP 2 385 043 B1**
(45) Date of publication and mention of the grant of the patent: **10.07.2013**
(21) Application number: 11164887.9
(22) Date of filing: 05.05.2011
(51) Int. Cl.: C07D 251/60, C07D 251/62

(54) **Low-energy-consumption process for the production of high-purity melamine, through the pyrolysis of urea and relative equipment**
Energiesparendes Verfahren zur Herstellung von hochreinem Melamin mittels Harnstoffpyrolyse und entsprechende Ausrüstung
Procédé de consommation basse énergie pour la production de mélamine à grande pureté, par la pyrolyse de l'urée et équipement associé

(30) Priority: 06.05.2010 IT IT20100810
(43) Date of publication of application: 09.11.2011
(73) Proprietor: EUROTECNICA MELAMINE, Luxembourg Zweigniederlassung in Ittigen, 3063 Ittigen (CH)
(72) Inventor: Borrini, Pietro, I-20090 Segrate-Milano (IT); De Amicis, Alberto, I-20097 San Donato Milanese (MI) (IT); Morello, Giovanni, I-20133 Milano (IT); Pichetta, Giulia, I-20136 Milano (IT); Santucci, Roberto, I-21050 Gorla Maggiore- Varese (IT); Tognoni, Antonio, I-20124 Milano (IT)
(74) Representative: De Gregori, Antonella

(56) References cited:
- WO-A1-03/095516
- US-B1- 6 252 074
- US-B2- 7 125 992

## Description

The present invention relates to a low-energy-consumption process for the production of high-purity melamine, through the high pressure pyrolysis of urea, and the relative equipment.

More specifically, the invention relates to a process which comprises the collection and purification in aqueous solution of the melamine produced in the pyrolysis reactor, and its separation by crystallization.

It is known that the transformation of molten urea into molten melamine is described by the following overall reaction (1):

6 NH₂CONH₂ → (CN)₃(NH₂)₃ + 6 NH₃ + 3 CO₂ urea melamine (1)

according to which, for each kilogram of molten melamine 1.86 kg of NH₃ and CO₂ are formed, called as a whole off-gas.

One of the most widespread industrial processes based on the pyrolysis of urea at high pressure, is that described in patent US 3,161,638 in the name of Allied. In this process, the whole biphasic effluent coming from the melamine synthesis reactor is cooled and collected in an aqueous ammonia medium.

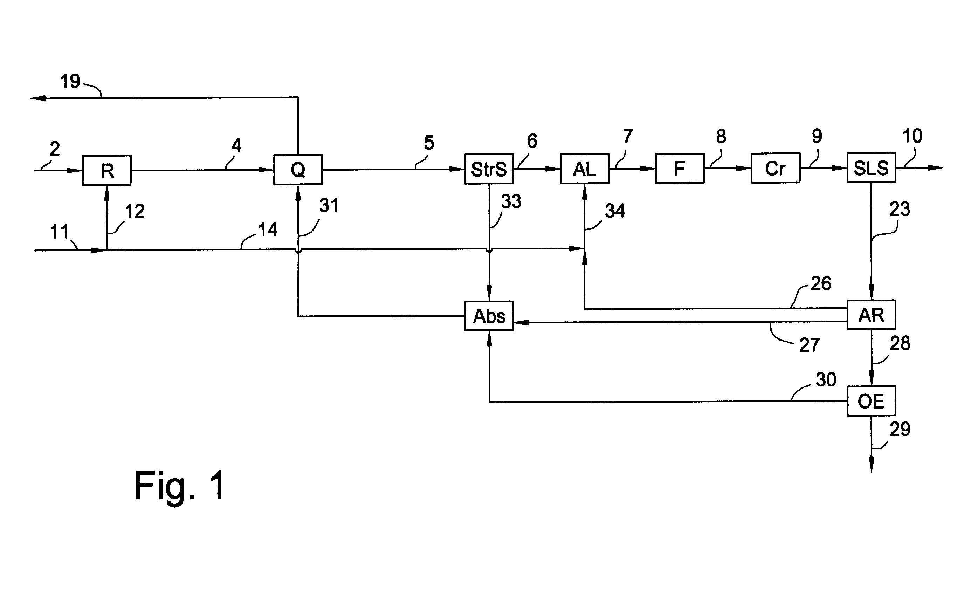

For a detailed illustration of the process according to patent US 3,161,638 mentioned above and in order to appreciate the improvements and advantages provided by the present invention, fig. 1 shows the simplified block scheme of an embodiment of the above process according to the state of the art.

According to the scheme of figure 1, the urea (stream 2) produced in an adjacent synthesis plant (not shown in figure 1) is sent in the liquid state, at a temperature of 135-145 °C, to a reaction section R consisting of a pyrolysis reactor where an appropriate heating system maintains the reagent system at a temperature of about 360-420 °C; the pressure is kept at a value higher than 70 barᵣₑₗ. Anhydrous gaseous NH₃ (stream 12) is also preferably introduced into the reactor together with the molten urea. The reactor has one single step and the reagent mass is maintained under strong circulation by the gases formed during the pyrolysis of the urea.

The whole reacted mass, consisting of a biphasic liquid/gas effluent (stream 4), is discharged into a quench section Q where, by contact with an aqueous ammonia solution (stream 31), its temperature is lowered to about 160°C. Under the above operating conditions, all the melamine, the non-reacted urea and various impurities formed during the synthesis (for example oxyaminotriazines OATs and polycondensates) pass into solution and are sent to the subsequent treatment (stream 5), whereas the remaining gaseous phase, substantially consisting of NH₃ and CO₂ coming from the reactor and saturation water vapour, is separated and sent to the urea plant (damp off-gas stream 19), after undergoing possible treatment (not indicated in figure 1), such as for example, condensation by absorption in aqueous solution.

The stream 5 contains a certain quantity of dissolved NH₃ and CO₂, which are eliminated in the subsequent stripping section with vapour StrS. The elimination of the CO₂ is necessary for obtaining a high purity of the melamine in the subsequent treatments; the elimination of the NH₃ is not necessary, but takes place due to the nature of the liquid/gas equilibria in the water - NH₃ - CO₂ system.

Two streams leave the StrS section: a gaseous stream (stream 33) comprising the NH₃ and CO₂ extracted from stream 5; and an aqueous stream comprising melamine and the remaining impurities (stream 6).

The stream 33 is sent to an absorption section Abs where it comes into contact with an aqueous stream 30, forming an aqueous stream 31 comprising the CO₂ and NH₃ extracted from stream 5. The aqueous stream 31 is in turn sent to the quench section Q, where, as already mentioned, it is used for cooling and dissolving stream 4 leaving the reactor.

The aqueous stream leaving the bottom of the section StrS (stream 6) contains residual CO₂ for about 0.3-0.5 % in mass, melamine for about 6-12% in mass, impurities of OATs and polycondensates and also the urea leaving the reactor and non-hydrolyzed to NH₃ and CO₂ in the quench Q and in the stripping StrS. This urea hydrolyzes in the sections of the plant downstream of the stripping StrS, as far as the deammoniation section AR (see further on), leading to the undesired formation of additional CO₂.

Due to their low solubility, the polycondensates must be eliminated before sending said stream 6 to the crystallization section Cr for the recovery of the melamine.

In order to eliminate the polycondensates, NH₃ (stream 34) is added to stream 6 until 12-15% in mass is reached. The resulting stream remains at about 170°C in an ammonolysis section AL, in an apparatus called "am*monolyser",* where the polycondensates are almost totally eliminated transforming most of them to melamine.

The aqueous ammonia solution leaving the section AL (stream 7) is sent to the finishing filtration section F and then to the crystallization section Cr (stream 8), where the temperature is lowered to about 40-50°C and most of the melamine crystallized. The high concentration of NH₃ and low concentration of CO₂ in the crystallization allow the OATs, whose solubility in a base increases considerably with the pH increase, to be maintained in solution, thus separating a high-purity product (higher than 99.8% in mass). The control of the CO₂ concentration in the crystallization is therefore a critical point of the process.

The aqueous ammonia suspension comprising the crystallized melamine leaving the section Cr (stream 9) is sent to the solid/liquid separation section SLS, where the crystallized melamine (stream 10) and a stream of mother liquor (stream 23) comprising OATs - formed during the pyrolysis reaction and also deriving from the hydrolysis of melamine in the various apparatuses where it has resided in warm aqueous phase - are separated from stream 9.

The stream of mother liquor 23, where the residual melamine is present at a concentration of 0.8 - 1% in mass, cannot be directly recycled to the quench section Q: this direct recycling would cause an increase in the concentrations of OATs, exceeding the saturation concentration in crystallization, where it would precipitate together with the melamine, polluting the product. The mother liquor stream 23, on the other hand, cannot be discharged directly into the environment due to environmental and economical problems linked to the strong content of NH₃ and organic compounds, among which melamine.

In order to overcome the problems connected to the direct recirculation of the crystallization mother liquor, the process according to patent US 3,161,368 envisages the deammoniation of the mother liquor in a section AR where three streams are separated by distillation: a stream of NH₃ substantially free of CO₂, to be recycled to the ammonolysis section AL (stream 26); a stream rich in CO₂, which is recycled to the absorber Abs (stream 27); an aqueous stream almost free of NH₃ and practically comprising only melamine and OATs (stream 28).

The stream 28 of deammoniated mother liquor is sent to a section OE for the elimination of the OATs and to obtain an aqueous solution to be recycled to the quench section Q (stream 30). The section OE can be obtained in two different ways, both applied in the state of the art:
a) in one case, the OATs are crystallized by cooling and neutralization with CO₂, and then separated from the stream 28 by ultra-filtration. This separation provides:
   - an aqueous stream 30 substantially free of OATs and rich in melamine, to be recycled to the quench Q (through the absorber Abs) thus also recovering the melamine contained therein;
   - a stream rich in OATs in suspension, to be sent to decomposition to recover the organic compounds in the form of NH₃ and CO₂, obtaining a stream of water (stream 29) substantially free of impurities, which can be discharged or re-used in a suitable point of the plant, for example instead of demineralized water;
b) in the other case, the whole stream 28 is sent to decomposition to recover the organic compounds in the form of NH₃ and CO₂, obtaining a stream of water (stream 29) substantially free of impurities, which can be discharged or re-used in a suitable point of the plant, and a stream of water 30 to be recycled to the quench Q (through the absorber Abs).

The process illustrated above is currently applied industrially in numerous plants, but is jeopardized by a high consumption of raw materials and utilities, such as for example, vapour, cooling water, fuel gas, electric energy). In particular, there is a high vapour consumption, due to the vapour stripping StrS and to the treatment of all the mother liquor in the deammoniation AR by distillation.

The solution of some of the drawbacks of the state of the art described above is contained in patent US 7,125,992 in the name of the same Applicant, which envisages a decrease in energy consumptions, investment and specific consumption of urea necessary for the production of melamine.

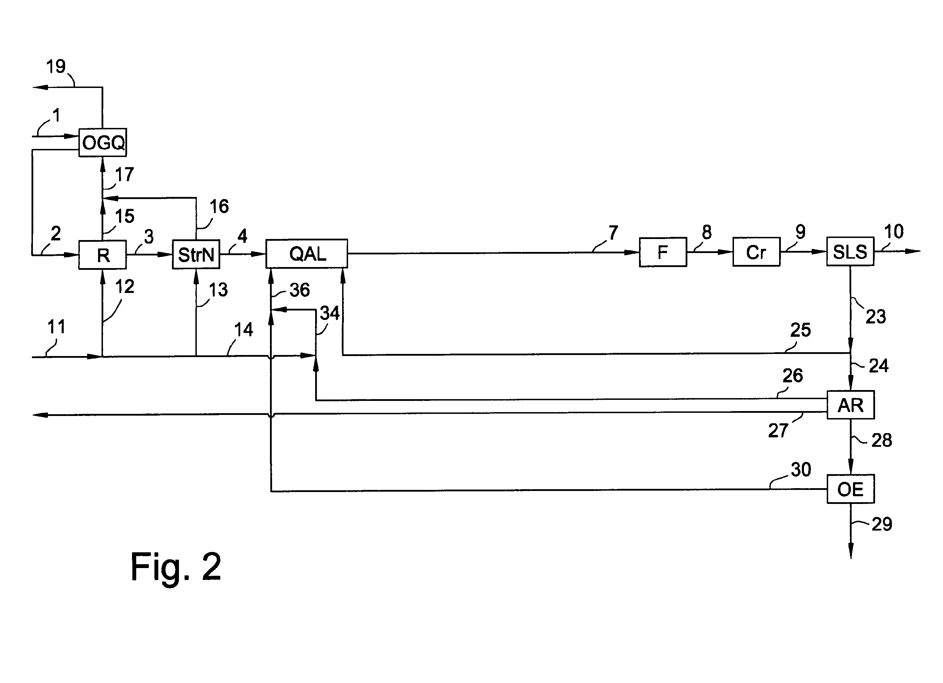

The process according to US 7,125,992 is illustrated in figure 2, where the streams and sections corresponding to those of the scheme of figure 1 are identified with the same numbers or abbreviations as figure 1 and will not be further described.

Unlike the state of the art represented by the process according to US 3,161,368, the process according to US 7,125,992 can be distinguished by:
a) the introduction of a stripping StrN upstream rather than downstream of the quench Q;
b) the use of NH₃ and not vapour in said stripping;
c) the separation of the anhydrous off-gas from the molten melamine in both the reaction (stream 15) and stripping (stream 16);
d) the introduction of a washing section OGQ of the anhydrous off-gas, coming from the reaction and stripping, by means of molten urea (stream 1), to completely recover the melamine vapour contained therein and to recycle it to the reaction R (stream 2).

The stripping StrN eliminates the CO₂ present in the raw melamine leaving the reaction through anhydrous gaseous NH₃ (stream 13); said NH₃ also favours the completion of the pyrolysis reaction, increasing the yield and eliminating the residual urea which would hydrolyze downstream giving further CO₂.

With respect to the process according to US 3,161,368, the process described in US 7,125,992 offers the following advantages:
a) eliminating the stripping of CO₂ with vapour, operating in aqueous phase (section StrS in figure 1), and also the absorption connected with this (section Abs of figure 1), with a considerable saving of vapour;
b) obtaining in a single quench-ammonolysis section (section QAL, figure 2) both the dissolution of the molten melamine and the transformation of most of the polycondensates into melamine. This section is fed by an aqueous ammonia solution (stream 36); furthermore, a part of the crystallization mother liquor (stream 25) is fed directly to the same.

In the process described in US 7,125,992, there is the necessity of recovering the melamine vapour contained in the anhydrous off-gas, before sending the latter to the urea plant. The concentration of melamine vapour in the off-gas is not negligible, possibly amounting to 2-5% in mass, depending on the operating conditions of the reactor and efficiency of the gas/liquid separation.

The above recovery is effected in the OGQ washing section using the same molten urea fed to the melamine plant (stream 1).

The use of the molten urea allows practically all of the melamine to be recovered and the anhydrous off-gas (stream 19) to be sent to a point of the urea plant which is at a compatible pressure. The molten urea which contains the melamine recovered is sent (stream 2) to the reactor.

Equipment for washing off-gas with molten urea is described, for example, in patents US 3,700,672 in the name of Nissan Chemical and US 4,565,867 in the name of Melamine Chemicals.

The OGQ section operates at approximately the same reaction pressure and temperatures generally around 180°C. The choice of the washing temperature derives from a compromise between the necessity of preventing the formation of solid ammonium carbamate from NH₃ and CO₂ and the necessity of limiting the degradation of the urea to undesired solid products (such as biuret, triuret or cyanuric acid). All these solid products cause considerable operative problems.

In the washing with molten urea, the condensable substances (essentially melamine) contained in the off-gas are condensed and solidified, remaining in solution/suspension in the molten urea. A significant dissolution of the same off-gas in the molten urea also takes place in parallel: data available in the state of the art indicate for example that the molten urea leaving the washing can contain 3-6% in mass of melamine in solution/suspension and about 20% in mass of off-gas in solution/emulsion. This generates an undesired recycling of the same off-gas towards the reactor, which, on the other hand, should be entirely sent to the urea plant.

Washing with urea consequent has various disadvantages:
a) the washing section with molten urea operates at practically the same pressure as the reactor and in the presence of CO₂, which, at the washing temperature, causes considerable corrosion also for stainless steels. In order to avoid this problem, resort must be made to more expensive materials, such as titanium or alloys of the Inconel and Hastelloy type;
b) the efficiency of the reactor is substantially reduced due to the recycling of both the melamine recovered (which is recycled in the reactor for about 5-10% of the quantity produced in the plant) and the off-gas (which is recycled for about 30-40% of the net quantity produced in the reaction). These recyclings cause a reduction in the volume available for the synthesis reaction, they disturb the circulation of the reagent mass and reduce the thermal exchange due to the increased gas/liquid ratio; a substantial increase in the thermal exchange surfaces is therefore necessary, with the same net production of melamine;
c) the reliability of the overall system formed by the reaction section and washing section with molten urea is reduced. A malfunctioning in the washing section, which functions under extremely delicate compromise conditions, generally requires the immediate stoppage of the reaction section and consequently the interruption of the production. Already in the state of the art (figure 1), stoppages of the reaction section (either programmed or accidental) imply complex procedures such as, for example, the emptying of the reactor, which is generally effected by sublimation of the melamine by the injection of gaseous NH₃ at high temperature. With washing with molten urea, the emptying procedure by sublimation becomes much more complex and costly, due to the urea which is still present in the washing section and which can no longer be sent to the reactor which has already been stopped. The plant must therefore be equipped with a specific additional section which receives, only in the above eventuality, the sublimation products;
d) in the deammoniation section AR, a stream of CO₂ comprising NH₃ and water is produced (stream 27), which cannot be joined with the anhydrous off-gas leaving the washing with molten urea, and which is therefore recovered at the urea plant as a separate stream. This stream sends water to the urea plant, partly attenuating the advantage associated with the sending of anhydrous off-gas.

An objective of the present invention is to overcome the drawbacks indicated in the state of the art.

A first object of the present invention relates to a low-energy-consumption process for the production of high-purity melamine, through the pyrolysis of urea, comprising the following operative steps:
a) separating a biphasic liquid-gas effluent product in a pyrolysis reaction of urea into a liquid stream of raw melamine and a first stream of anhydrous off-gas comprising NH₃, CO₂ and melamine vapour;
b) putting the above liquid stream of raw melamine in contact with a stream of gaseous anhydrous NH₃ and forming a liquid stream of raw melamine impoverished in CO₂ and a second stream of anhydrous off-gas comprising NH₃, CO₂ and melamine vapour;
c) putting the above first and second anhydrous off-gas streams in contact with at least one aqueous washing stream and forming an aqueous stream comprising melamine, NH₃, CO₂ and a stream of damp off-gas comprising NH₃, CO₂ and water vapour;
d) removing from said aqueous stream comprising melamine, NH₃, CO₂, at least a part of the CO₂ contained therein, and forming a stream comprising the CO₂ removed and an aqueous stream comprising melamine and impoverished in CO₂;
e) recovering the melamine contained in said liquid stream of raw melamine impoverished in CO₂ and the melamine contained in said aqueous stream comprising melamine and impoverished in CO₂ through crystallization by cooling, with the formation of a stream of crystallized melamine and a stream of mother liquor.

A second object of the present invention relates to the equipment for applying the above process, comprising:
i) a separation section for separating a liquid/gas biphasic effluent produced in a pyrolysis reaction of urea into a liquid stream of raw melamine and a first stream of anhydrous off-gas comprising NH₃, CO₂ and melamine vapour, said separation section being connected to a reaction section for the pyrolysis of urea from which it receives said biphasic liquid/gas effluent, said separation section being inside or outside said reaction section;
ii) a stripping section for putting said liquid stream of raw melamine coming from said reaction section in contact with an anhydrous gaseous stream of NH₃ and forming a liquid stream of raw melamine impoverished in CO₂ and a second stream of anhydrous off-gas comprising NH₃, CO₂ and melamine vapour, said stripping section being connected to said reaction section from which it receives said liquid stream of raw melamine;
iii) a washing section for putting said first and second streams of anhydrous off-gas in contact with at least one aqueous washing stream and forming an aqueous stream comprising melamine, NH₃ and CO₂ and a stream of damp off-gas comprising NH₃, CO₂ and water vapour, said washing section being connected to said separation section from which it receives said first stream of anhydrous off-gas and to said stripping section from which it receives said second stream of anhydrous off-gas;
iv) a removal section of CO₂ for removing, from said aqueous stream comprising melamine, NH₃ and CO₂, at least a part of the CO₂ contained therein, and forming a stream comprising the CO₂ removed and an aqueous stream comprising melamine and impoverished in CO₂, said CO₂ removal section being connected to said washing section from which it receives said aqueous stream comprising melamine, NH₃ and CO₂;
v) at least one melamine recovery section for recovering both the melamine contained in said liquid stream of raw melamine impoverished in CO₂ and the melamine contained in said aqueous stream comprising melamine and CO₂ through cooling crystallization, with the formation of a stream of crystallized melamine and a stream of mother liquor, said recovery section of the melamine being connected to both the stripping section from which it receives said liquid stream of raw melamine impoverished in CO₂ and said CO₂ removal section from which it receives said aqueous stream comprising melamine and impoverished in CO₂.

In its essence, the process, object of the present invention, envisages separately treating the two components of the biphasic liquid/gas effluent which is generated during the pyrolysis reaction of urea, i.e. the gaseous phase consisting of anhydrous off-gas and the liquid phase consisting of raw melamine.

In particular, the separate removal of the CO₂ from the anhydrous off-gas and CO₂ from the raw melamine requires a much lower energy consumption with respect to the removal of CO₂ from the whole biphasic effluent collected in an aqueous ammonia solution as is the case in the state of the art (figure 1).

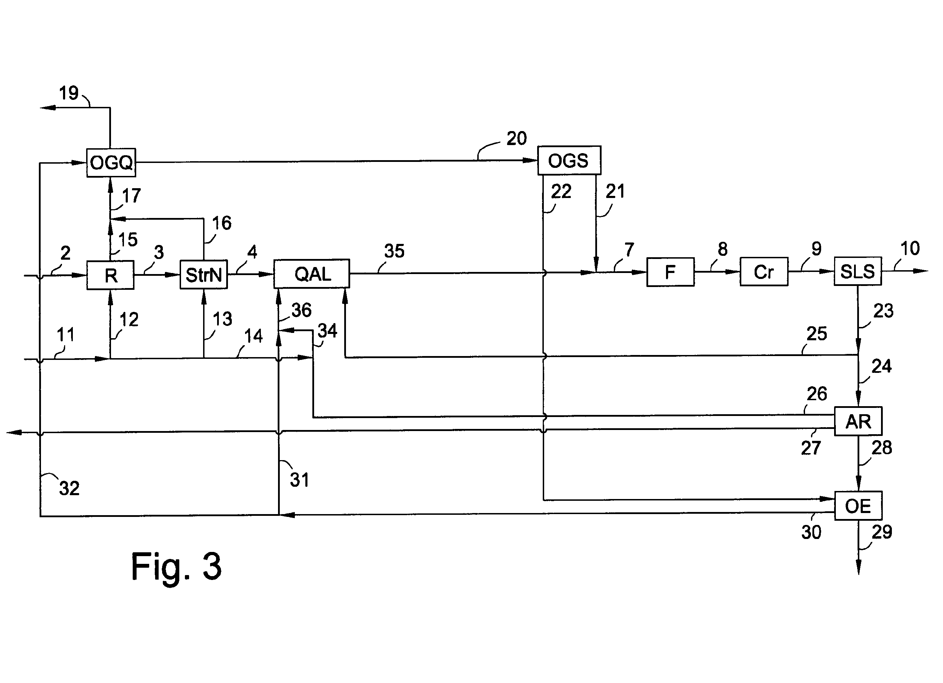

The process according to the present invention and the advantages deriving therefrom can be better understood from the following description of one of its embodiments, illustrated in figure 3.

The description of this embodiment and relative process scheme should not be considered as limiting the protection scope defined by the enclosed claims.

The block diagram of fig. 3 shows the main sections of a melamine production plant and the main material streams according to the process of the present invention.

A stream 2 of liquid urea at a temperature higher than the melting point (equal to about 133°C) and a stream 12 of gaseous, anhydrous NH₃ are sent to a reaction section R. The section R comprises a reactor equipped with a suitable heating system which maintains the reagent system at a temperature of about 360-420 °C; the pressure is maintained at a value higher than 70 bar_{rel..}

Inside the reactor, or in one or more separators positioned downstream, (not shown in figure 3), or again in the stripping section with NH₃ (StrN) downstream of the reactor (described hereunder), the biphasic liquid/gas effluent produced by the urea pyrolysis reaction, is separated in a liquid stream 3 of raw melamine comprising non-reacted urea, NH₃, CO₂ and impurities such as OATs and polycondensates and a first stream of anhydrous off-gas 15, comprising NH₃, CO₂ and melamine vapour.

When the separation is effected in section StrN, phases a) and b) of the process are effected contemporaneously in this section, from which a single anhydrous off-gas stream exits, comprising NH₃, CO₂ and melamine vapour, which is sent to phase c) to be washed with water, and a stream of raw melamine impoverished in CO₂.

The liquid stream 3 of raw melamine and the stream 15 of anhydrous off-gas are subjected to two different types of treatment for the recovery of the melamine contained therein.

The liquid stream of raw melamine 3 is sent to a stripping section StrN, which preferably operates at the same temperature and pressure conditions as section R, wherein it is put in contact with a stream 13 of anhydrous, gaseous NH₃. The mass ratio between said stream 13 and said stream 3 ranges from 0.06 to 0.60, and is preferably equal to 0.20.

The anhydrous gaseous stream of NH₃ flows in close contact with the liquid stream of raw melamine and extracts the CO₂ dissolved therein, until a residual concentration of about 200 ppmw is reached, lower than that obtained in the stripping section StrS of the state of the art represented by the procedure of figure 1.

Furthermore, the residence of the raw melamine under the stripping conditions of CO₂ with NH₃ offers advantageous side-effects:
a. it allows the conversion of non-reacted urea to melamine to advance almost completely, leading to an increase in the yield and reducing the formation of CO₂ in the downstream sections, due to the decomposition of the residual urea;
b. it allows the conversion of OATs to melamine to advance, until about 6,000 ppmw or less of OATs are obtained at the outlet. The conversion degree of OATs to melamine depends on the residence time of the stream 3 in contact with the stream 13 in the section StrN;
c. by increasing the partial pressure of NH₃, it favours the re-conversion of the polycondensates to melamine, lowering their concentration by over 20%.

A liquid stream of melamine (stream 4) therefore leaves the section StrN, which is not only practically free of CO₂, but also partially purified as it is practically free of urea and contains reduced quantities of OATs and polycondensates. A second stream of anhydrous off-gas (stream 16) also leaves the section StrN, mainly comprising NH₃, a little CO₂ and a certain quantity of melamine vapour.

The anhydrous off-gas streams coming from the sections R and StrN are subjected, together or separately, to a recovery treatment of melamine by washing with water, in a single washing section OGQ or in separate washing sections (not represented in figure 3). The second anhydrous off-gas stream 16 is preferably joined to the first stream 15 of anhydrous off-gas, forming a single stream 17 of anhydrous off-gas to be subjected to the same treatment in a single washing section OGQ.

The stream 17 can contain up to 10% in mass of the total melamine vapour produced in the sections R and StrN. Said melamine is recovered in the section OGQ by putting the stream 17 in contact with an aqueous washing stream, preferably consisting of one or more streams collected from suitable points of the same melamine plant (in Fig 3 these streams are represented by a single stream 32), with the formation of aqueous stream (stream 20), comprising melamine, NH₃ and CO₂.

The section OGQ operates at temperatures ranging from 125-190°C, preferably 160-175°C, and pressures within the range of 20-30 bar_{rel.}, preferably at about 25 barᵣₑₗ; the mass ratio between the stream 32 and the stream 17 ranges from 0.3 to 2.0, preferably from 0.4 to 0.7.

Some possible ways for forming the washing section of the off-gas with aqueous solution are illustrated in the patents CN 1300122C and in the international patent application WO 03/095516 Al in the name of the Applicant, which specifically and exclusively relate to this operation.

The gaseous stream leaving the section OGQ after washing (stream 19) consists of damp off-gas substantially including NH₃, CO₂ and saturation water vapour; it is sent to the urea plant as such or after undergoing treatment (not shown in figure 3), such as, for example, condensation by absorption in aqueous solution.

The aqueous stream leaving the section OGQ (stream 20) is sent to a section OGS for the separation of CO₂, where a part of the CO₂ contained therein is removed, preferably by flash, and even more preferably by vapour stripping, produced, for example, by a reboiler at the bottom of the stripper itself. A gaseous stream rich in CO₂ (stream 22) is recovered from the section OGS and sent to a point of the process where it can no longer contribute to lowering the crystallization pH, together with an aqueous stream comprising melamine and impoverished in CO₂ (stream 21).

As already mentioned, the liquid stream 4 of melamine, substantially without CO₂, leaves the stripping section with NH₃, StrN. The same is sent to a quench-ammonolysis section QAL, where the quench (dissolution in water of the raw melamine) and ammonolysis (elimination of the polycondensates) treatments are effected, corresponding to the treatments effected in the sections Q and AL, respectively, of the state of the art (figure 1).

The section QAL can consist of one or more stationing apparatuses, preferably a single apparatus. The stream 4 enters the bottom of said equipment, maintained under vigorous stirring, and is put in close contact with an aqueous ammonia solution (stream 36), where it is completely dissolved at a temperature of 160-180°C, preferably 170-172°C. The stirring and contact between streams 4 and 36 can be obtained by means of distributors, static mixers, fillings, internal stirrers, external circulation pumps or any other system normally used in chemical industry for favouring the complete mixing of various fluid streams.

The aqueous stream 36 is formed by the joining of an aqueous stream (stream 31) coming from the subsequent phases of the melamine process and a stream of NH₃ (stream 34), formed, in turn, by the joining of a stream of NH₃ 26 also coming from the subsequent phases of the melamine process and the make-up stream of NH₃ 11; a direct recycling aqueous stream of the crystallization mother liquor (stream 25) is also sent to the quench-ammonolysis section.

The mass ratios, at the inlet of the quench-ammonolysis, between the aqueous streams 31, 34, 25 and the stream of molten melamine 4, are selected so as to have at the exit, in the aqueous ammonia solution comprising purified melamine (stream 35), concentrations of NH₃ ranging from 10 to 17% in mass (preferably from 12 to 15% in mass) and concentrations of melamine ranging from 5 to 19% in mass (preferably from 7 to 15% in mass, more preferably from 9 to 11% in mass).

The residence time in the quench-ammonolysis equipment is sufficient for almost completely eliminating the polycondensates present in the stream 4, transforming most of them into melamine. As far as the OATs are concerned, the unification of the quench and ammonolysis sections and the suppression of the intermediate vapour stripping section (section StrS in figure 1) reduce the residence time of the melamine in aqueous phase under heat, causing a lower formation of OATs through hydrolysis (from about 10 to about 20% less with respect to the state of the art represented in figure 1, according to the reduction degree applied to the various residence times in the various apparatuses).

The stream 21 coming from the section OGS, is preferably joined with the stream 35 leaving the quench-ammonolysis to form the stream 7; from this point on, the melamine leaving the reaction section R and the stripping section with NH₃ StrN in vapour phase, joins the melamine which has left the same sections in liquid phase, and undergoes the same treatment.

We note incidentally that the stream 21 comprising the melamine coming, as vapour, from the reaction and stripping with NH₃, can also have different destinations with respect to the destination illustrated in figure 3:
a) in a first variant (not shown in figure 3) the stream 21 can be reintroduced into the main circuit (joining the melamine from liquid phase) more downstream with respect to the point indicated in figure 3, being separately subjected to specific filtration treatments, and possibly also crystallization treatments, and possibly also solid/liquid separation of the crystallized melamine from the aqueous solution in which it is dispersed;
b) in a second variant (also not shown in figure 3), the melamine contained in the stream 21 can be kept separate until the final product step, taking advantage of its different composition and in particular the lower content of impurities with respect to the stream 35.

The stream 7 is sent to the filtration section F, consisting of finishing filters which also complete the ammonolysis treatment. The stream leaving the section F (stream 8) is sent to the crystallization section Cr, where the melamine is precipitated by lowering the temperature to a value of about 40 - 50°C, obtaining an aqueous suspension of high purity melamine (stream 9).

The low content of CO₂ in the stream 21 obtained by means of the section OGS and the low content of CO₂ in the stream 35 obtained through the section StrN, allow the crystallization pH to be kept high, thus operating under more unfavourable conditions for the precipitation of OATs. Unlike the state of the art (figure 1), these advantageous conditions for the crystallization of pure melamine are obtained: in the section OGS, by separating the CO₂ not from the whole stream of melamine produced (stream 5, figure 1), but only from the stream of melamine recovered from the off-gas (stream 20, figure 3), which is quantitatively much less; in the section StrN, by stripping the CO₂ without using vapour as in the section StrS (figure 1), but using only NH₃ coming from the urea plant (stream 13, figure 3) and being returned to this urea plant with the damp off-gas (stream 19 of figure 3). Furthermore, in the section StrN, only CO₂ is removed, whereas, as already mentioned, in the section StrS, the NH₃ must also be removed.

The stream 9 leaving the section Cr is subjected to a solid/liquid separation in the section SLS, where the melamine crystals (stream 10) are separated from the crystallization mother liquor (stream 23) and sent to the drying and packaging sections (not indicated in figure 3).

As already mentioned, thanks to the lower residence time of the melamine in hot aqueous phase, the stream 23 of mother liquor contains a lower quantity of OATs with respect to the corresponding stream 23 in the process of the state of the art represented in figure 1; it can therefore be partially recycled directly to the quench-ammonolysis section QAL without any treatment, in such a quantity as to remain in any case below the saturation of the OATs in the crystallization.

The stream 23 leaving the separator SLS is therefore divided into two streams (streams 24 and 25): stream 25 is recycled directly to the quench-ammonolysis section QAL, whereas stream 24 is sent to the deammoniation section AR.

The lower the quantity of OATs in the mother liquor, the greater the fraction of mother liquor which can be recycled directly to the section QAL, and the greater the savings on the vapour consumption and investment in the section AR and the direct recovery of melamine in the section QAL will be. With the process according to the present invention, the amount of mother liquor which can be recycled directly to the section QAL (stream 25) is 10% higher than the total amount of mother liquor (stream 23); it is preferably 20% higher.

The fraction of mother liquor which cannot be recycled directly (stream 24) is subjected to deammoniation in the section AR, which separates three streams: a stream of NH₃ substantially free of CO₂, to be recycled to the ammonolysis section QAL (stream 26); a stream rich in CO₂ (stream 27); an aqueous stream comprising melamine, OATs and substantially free of CO₂ and NH₃ (stream 28 of deammoniated mother liquor). The deammoniation AR can operate with any known method for the separation of water-NH₃-CO₂ mixtures.

The stream of NH₃ 26 is extracted from the section AR preferably in the gaseous state and then mixed with a part of the aqueous stream 31 (not shown in figure 3); in this way, the condensation enthalpy of the NH₃ heats the resulting stream, with a positive effect on the thermal balance of the section QAL and consequently on the vapour consumption of the whole plant. The recovery of the condensation heat of the NH₃ cannot be effected in the state of the art (figure 1), where the stream 26 goes to the section AL which does not need the above contribution to its thermal balance.

The stream rich in CO₂ 27 can be sent directly to the urea plant (figure 3), or recycled to a point in the melamine plant; it is preferably recycled to a point where the CO₂ contained therein cannot lower the crystallization pH, and can ultimately leave the melamine plant, taking less water with it than if it were sent directly to the urea plant (for example, after treatment which reduces its water content).

The stream of deammoniated mother liquor 28 is sent to a section OE to eliminate the OATs and obtain an aqueous solution to be recycled to the quench-ammonolysis section QAL (stream 31) and off-gas washing section OGQ (stream 32).

The section OE can be obtained in many different ways. The two preferred ways are already applied in the state of the art and are the following:
a) in one case, the OATs are crystallized by cooling and neutralization with CO₂, and then separated from the stream 28 by ultrafiltration. This separation provides:
   - an aqueous stream 30 substantially free of OATs and rich in melamine, to be recycled to the sections QAL and OGQ (streams 31 and 32) thus also recovering the melamine contained therein;
   - a stream rich in OATs in suspension, to be sent for decomposition to recover the organic compounds in the form of NH₃ and CO₂, obtaining a stream of water (stream 29) substantially free of impurities, which can be discharged or re-used in a suitable point of the plant, for example instead of demineralized water;
b) in the other case, the whole stream 28 is sent for decomposition to recover the organic compounds in the form of NH₃ and CO₂, obtaining a stream of water (stream 29) substantially free of impurities, which can be discharged or re-used in a suitable point of the plant, and a stream of water 30 to be recycled to the sections QAL and OGQ (streams 31 and 32).

The process according to the present invention provides the following advantages, relating to the consumption of utilities (especially energy as vapour and cooling water), investment and urea consumption:
a) the elimination of vapour stripping of CO₂ from the main line (section StrS, figure 1) and consequent absorption of the vapours obtained (section Abs, figure 1) considerably reduces the consumption of utilities;
b) the direct recycling of part of the mother liquor coming from the solid/liquid separation SLS to the quench-ammonolysis section QAL, with a consequent reduction in the amount of mother liquor to be treated in the deammoniation AR, allows a further reduction in the consumption of utilities;
c) the condensation to mixtures of gaseous NH₃ (stream 26) leaving the deammoniation AR improves the thermal balance of the quench-ammonolysis QAL, with a further reduction in the consumption of utilities. The reduction in the specific consumption of vapour due to points a), b) and c) is equal to about 60% with respect to the state of the art (figure 1); the corresponding reduction in the specific consumption of cooling water is equal to about 55%;
d) the purification in aqueous phase of the melamine is considerably simplified, with the above-mentioned elimination of the vapour stripping of CO₂ and absorption connected therewith, and with the unification of the quench Q and ammonolysis AL (figure 1) in the quench-ammonolysis section QAL (figure 3), which is also obtained with less costly materials with respect to the quench Q;
e) the dimensions of the deammoniation AR and subsequent treatment of the mother liquor OE are reduced. The investment reduction due to points d) and e) is higher than the increase due to the introduction of the stripping StrN, washing of the off-gas OGQ and removal of the stream OGS; the overall reduction in the investment is estimated at about 10% with respect to the state of the art (figure 1);
f) the lesser hydrolysis of the melamine to OATs during the purification, thanks to the reduction in the total volume where the melamine remains in hot aqueous solution, leads to an increase in the yield;
g) the almost total conversion of the urea and transformation of part of the OATs into melamine in the stripping with NH₃ leads to a further increase in the yield. The reduction in the specific consumption of urea due to points f) and g) is higher than 5% with respect to the state of the art (figure 1);
h) the washing of the anhydrous off-gas with aqueous solution allows a simple and effective recovery of all the melamine vapour, with blander operating conditions and more economical construction materials with respect to washing with molten urea (figure 2);
i) the recovery of the melamine vapour by washing with aqueous solution allows avoiding problems associated with the recycling to the reaction of molten urea comprising melamine in solution/suspension and off-gas in solution/emulsion (figure 2);
j) the process can also be applied to plants constructed according to the state of the art, such as, for example, those necessary for effecting the processes schematically represented in figures 1 and 2, by making modifications which are not excessively complex (socalled revamping).

The following embodiment example is provided for purely illustrative purposes of the present invention and does not limit its protection scope defined by the enclosed claims.

### EXAMPLE

16.0 t/h of molten urea and 1.0 t/h of gaseous NH₃ are sent to the reactor in a melamine plant having a nominal capacity of 40,000 t/y; the reactor operates at 380°C and 80 barᵣₑₗ.
11.8 t/h of anhydrous off-gas comprising melamine and 5.2 t/h of liquid raw melamine, are separated from the reactor.

Said liquid melamine is treated under the same conditions as the reactor in a stripper with 1.1 t/h of anhydrous gaseous NH₃, obtaining 5.1 t/h of liquid melamine comprising only 0.02% in mass of dissolved CO₂; this stream also contains 3.4% in mass of polycondensates, 0.6% in mass of OATs and is substantially free of non-reacted urea.
1.2 t/h of anhydrous off-gas comprising melamine vapour are separated from the stripper, which, when joined with the off-gas leaving the reactor, form a stream of 13.0 t/h comprising 3.8% in mass of melamine vapour.

The liquid melamine leaving the stripper is sent to the quench-ammonolyser for purification in aqueous solution with NH₃, under operative conditions (temperature 172°C, pressure 25 barᵣₑₗ and concentration of NH₃ of 14% in mass) which are as such as to allow the almost total elimination of the polycondensates, mostly transformed into melamine.
63.0 t/h of an aqueous solution at 8% in mass of melamine, comprising about 3,000 ppm in mass of OATs and about 800 ppm in mass of polycondensates, leave the quench-ammonolyser.

The gaseous stream obtained by joining the anhydrous off-gas streams from the reactor and stripper, is sent to a washing column which operates at 169°C and 25 barᵣₑₗ, where it is put in contact in countercurrent with 7.0 t/h of an aqueous recycling solution.

The washed off-gas leaving the head of the washing column, containing 18% in mass of water and substantially free of melamine, is sent to the adjacent urea plant for the recovery of the NH₃ and CO₂ contained therein.
6.4 t/h of an aqueous solution comprising 0.5 t/h of melamine entering as vapour with the off-gas, and 4.5% in mass of CO₂, leave the bottom of the washing column. This stream is sent to a vapour stripping column, which operates at the bottom at 160 °C and 7 barᵣₑₗ. 4.6 t/h of an aqueous solution leave the bottom of the stripping column, where the CO₂ is reduced to 0.2% in mass.

This solution is joined to that leaving the quench-ammonolysis section, and the whole mixture is sent to filters from which a solution with less than 100 ppm in mass of polycondensates exits; the latter solution is then sent to the crystallizer, operating at a temperature of 45 °C, a pressure of 0,5 barᵣₑₗ, a concentration of CO₂ of about 0.15% in mass and a pH of about 11.5.

The suspension leaving the crystallizer is sent to a solid/liquid separator (centrifuge), which separates the crystallization mother liquor from the high-purity melamine (titre of over 99.9% in mass with respect to the dry product).

The approximately 62.6 t/h of mother liquor are divided into two streams. One stream of 7.5 t/h is recycled directly to the quench-ammonolysis section, without undergoing any treatment; the remaining 55.1 t/h are sent to the deammoniation section for the recovery of the NH₃ dissolved therein.

The deammoniation section is composed of a distillation column, from whose head almost anhydrous gaseous NH₃ exits. A liquid stream of CO₂, NH₃ and water leaves an intermediate plate of the column, and it is recycled to a suitable point of the process.

The aqueous solution leaving the bottom of the column is sent for treatment to eliminate the OATs by thermal decomposition alone. In this way, an aqueous solution is separated, which is recycled to the quench-ammonolysis and off-gas washing, together with a further aqueous solution which can be discharged or re-used as water for the utilities (for example make-up water for the cooling towers) or as process water.

A part of the solution recycled to the quench-ammonolysis section is mixed with gaseous NH₃ leaving the head of the distillation column of the deammoniation section, condensing it and recovering its condensation heat.

The consumptions of urea and vapour of the whole process prove to be respectively 3.2 t/t and 4.3 t/t of melamine produced, against the corresponding values of 3.4 t/t and 12.5 t/t necessary with the process of the state of the art represented in figure 1. If the elimination of the OATs takes place by ultrafiltration followed by decomposition of the retentate, the lower decomposition of melamine lowers the consumptions of urea to 3.05 t/t and 3.2 t/t (instead of 3.2 t/t and 3.4 t/t), for the process, object of the invention, and for the state of the art of figure 1, respectively.

The higher efficiency of the process according to the present invention derives in particular from the choice of eliminating the CO₂ present in the reaction effluent by separate treatment of the anhydrous off-gas and raw melamine. The separate treatment, in fact, makes it possible to operate in crystallization at the same pH of about 11.5 with respect to the process of figure 1, but consuming much less vapour to remove the CO₂, to specifically keep its concentration low in crystallization. A further advantage is provided by the possibility of recycling about 12% of the mother liquor stream leaving the solid/liquid separation section directly to the quench-ammonolysis section.

## Claims

1. A low-energy consumption process for the production of high-purity melamine, through the pyrolysis of urea, comprising the following operative steps:
a) separating a biphasic liquid-gas effluent product in a pyrolysis reaction of urea in a liquid stream of raw melamine (**3**) and a first stream of anhydrous off-gas (**15**) comprising NH₃, CO₂ and melamine vapours;
b) putting said liquid stream of raw melamine (**3**) in contact with a stream of gaseous anhydrous NH₃ (**13**) and forming a liquid stream of raw melamine impoverished in CO₂ (**4**) and a second stream of anhydrous off-gas (**16**) comprising NH₃, CO₂ and melamine vapours;
c) putting said first and second anhydrous off-gas streams (**15, 16**) in contact with at least one aqueous washing stream (**32**) and forming an aqueous stream (**20**) comprising melamine, NH₃, CO₂ and a stream of damp off-gas (**19**) comprising NH₃, CO₂ and water vapour;
d) removing from said aqueous stream (**20**) comprising melamine, NH₃, CO₂, at least a part of the CO₂ contained therein, and forming a stream (**22**) comprising the CO₂ removed and an aqueous stream (**21**) comprising melamine and impoverished in CO₂;
e) recovering the melamine contained in said liquid stream (**4**) of raw melamine impoverished in CO₂ and the melamine contained in said aqueous stream (**21**) comprising melamine and impoverished in CO₂ through crystallization by cooling, with the formation of a stream (**10**) of crystallized melamine and a stream (**23**) of mother liquor.

2. The process according to claim 1, **characterized in that** before said liquid stream (**4**) of raw melamine impoverished in CO₂ is subjected to crystallization by cooling, it is subjected to a quench-amonolysis treatment by contact with an aqueous ammonia stream (**36**)**,** with the formation of an aqueous ammonia stream (**35**) comprising purified melamine.

3. The process according to claim 2, **characterized in that** said aqueous stream (**21**) comprising melamine and impoverished in CO₂ is joined to said aqueous ammonia stream (**35**) comprising purified melamine, with the formation of a single stream (**7**) to be subjected to said step e).

4. The process according to any of the previous claims, **characterized in that** in step c) the removal of CO₂ from said aqueous stream (**20**) comprising melamine, NH₃ and CO₂ is effected by means of depressurization.

5. The process according to any of the claims 1-3, **characterized in that** in step d) the removal of CO₂ from said aqueous stream (**20**) comprising melamine, NH₃ and CO₂ is effected by means of stripping.

6. The process according to any of the previous claims, **characterized in that** said stream (**22**) comprising the removed CO₂ obtained in step d) is recycled to a step of the same process, subsequent to said step e) for the recovery of melamine through crystallization by cooling.

7. The process according to any of the previous claims, **characterized in that** said stream of damp off-gas (**19**) comprising melamine, NH₃, CO₂ and water vapour is recycled to a urea production plant, as such or after having been subjected to a condensation treatment by absorption in aqueous solution or other type of treatment.

8. The process according to claim 2, **characterized in that** at least a part of said stream of mother liquor (**23**) obtained in step e) is used as aqueous stream (**25**) in the quench-amonolysis treatment of said liquid stream of raw melamine impoverished in CO₂ (**4**)**.**

9. The process according to any of the previous claims, **characterized in that** at least a part of said stream of mother liquor (**23**) obtained in step e) is subjected to a deammoniation treatment, with the formation of a gaseous stream of NH₃, substantially free of CO₂ (**26**), a stream (**27**) comprising CO₂ and an aqueous stream (**28**) of deammoniated mother liquor.

10. The process according to the previous claim, **characterized in that** said stream (**27**) comprising CO₂ is recycled to a urea production plant, directly or after a treatment which reduces its water content.

11. The process according to claim 9 or 10, **characterized in that** said aqueous stream (**28**) of deammoniated mother liquor is subjected to a decomposition treatment with the recovery of NH₃ and CO₂, and the production of an aqueous stream which is partially recycled, as an aqueous washing stream (**32**), to said step c) and partially recycled as aqueous stream (**31**) to contribute to the formation of said aqueous ammonia stream (**36**) to said quench-amonolysis treatment.

12. The process according to claim 9 or 10, **characterized in that** said aqueous stream (**28**) of deammoniated mother liquor is separated into:
i) an aqueous stream enriched in melamine which is partially recycled as aqueous washing stream (**32**) to said step b) and partially recycled as aqueous stream (**31**) to contribute to the formation of said aqueous ammonia stream (**36**) to said quench-amonolysis treatment;
ii) an aqueous stream enriched in OATs, suitable for being decomposed with the recovery of NH₃ and CO₂.

13. The process according to claims 11 or 12, **characterized in that** said aqueous stream (**26**) of NH₃ substantially free of CO₂ is mixed with a part of said aqueous stream (**31**) to recover the condensation heat therefrom.

14. The process according to any of the previous claims, **characterized in that** said steps a) and b) are effected contemporaneously with the formation of a single stream of anhydrous off-gas comprising NH₃, CO₂ and melamine vapour and a stream of raw melamine impoverished in CO₂, said single stream of anhydrous off-gas being sent to step c).

15. Equipment for applying the improved process for the production of melamine as defined in claim 1, comprising:
i) a separation section for separating a liquid/gas biphasic effluent produced in a pyrolysis reaction of urea in a liquid stream of raw melamine (**3**) and a first stream of anhydrous off-gas (**15**) comprising NH₃, CO₂ and melamine vapour, said separation section being connected to a reaction section (**R**) for the pyrolysis of urea from which it receives said biphasic liquid/gas effluent, said separation section being inside or outside said reaction section (**R**);
ii) a stripping section (**StrN**) for putting said liquid stream of raw melamine coming from said reaction section (**R**) in contact with an anhydrous gaseous stream of NH₃ forming a liquid stream of raw melamine impoverished in CO₂ (**4**) and a second stream of anhydrous of off-gas (**16**) comprising NH₃, CO₂ and melamine vapour, said stripping section (**StrN**) being connected to said reaction section (**R**) from which it receives said liquid stream of raw melamine (**4**);
iii) a washing section **(OGQ)** for putting said first and second streams of anhydrous off-gas (**15, 16**) in contact with at least one aqueous washing stream (**32**) and forming an aqueous stream (**20**) comprising melamine, NH₃ and CO₂ and a stream of damp off-gas (**19**) comprising NH₃, CO₂ and water vapour, said washing section (**OGQ**) being connected to said separation section from which it receives said first stream of anhydrous off-gas (**15**) and to said stripping section (**StrN**) from which it receives said second stream of anhydrous off-gas (**16**);
iv) a removal section of CO₂ (**OGS**) for removing, from said aqueous stream (**20**) comprising melamine, NH₃ and CO₂, at least a part of the CO₂ contained therein, and forming a stream (**22**) comprising the CO₂ removed and an aqueous stream (**21**) comprising melamine and impoverished in CO₂, said CO₂ removal section (**OGS**) being connected to said washing section (**OGQ**) from which it receives said aqueous stream (**20**) comprising melamine, NH₃ and CO₂;
v) at least one melamine recovery section (**Cr**) for recovering both the melamine contained in said liquid stream of raw melamine impoverished in CO₂ (**4**) and the melamine contained in said aqueous stream (**21**) comprising melamine and CO₂ through cooling crystallization, with the formation of a stream (**10**) of crystallized melamine and a stream (**23**) of mother liquor, said recovery section (**Cr**) of the melamine being connected to both the stripping section (**StrN**) from which it receives said liquid stream (**4**) of raw melamine impoverished in CO₂ and said removal section of CO₂ (OGS) from which it receives said aqueous stream (21) comprising melamine and impoverished in CO₂.

## Patentansprüche

1. Energiesparendes Verfahren für die Herstellung von hochreinem Melamin durch Pyrolyse von Harnstoff, enthaltend die folgenden Arbeitsschritte:
a) Trennen eines zweiphasigen Flüssigkeit-Gas-Abflussprodukts in einer Harnstoff-Pyrolysereaktion in einen Rohmelamin-Flüssigkeitsstrom (3) und einen ersten Strom aus wasserfreiem Abgas (15), der NH₃, CO₂ und Melamindämpfe enthält;
b) Kontaktieren des Rohmelamin-Flüssigkeitsstroms (3) mit dem Strom aus gasförmigem wasserfreiem NH₃ (13) und Bilden eines CO₂-verarmten Rohmelamin-Flüssigkeitsstroms (4) und eines zweiten Stroms aus wasserfreiem Abgas (16), der NH₃, CO₂ und Melamindämpfe enthält;
c) Kontaktieren des ersten und des zweiten wasserfreien Abgasstroms (15, 16) mit mindestens einem wässrigen Waschstrom (32) und Bilden eines wässrigen Stroms (20), der Melamin, NH₃, CO₂ enthält, und eines feuchten Abgasstroms (19), der NH₃, CO₂ und Wasserdampf enthält;
d) Entfernen aus dem Melamin, NH₃, CO₂ enthaltenden wässrigen Strom (20) zumindest eines Teils des darin enthaltenen CO₂ und Bilden eines Stroms (22), der das entfernte CO₂ enthält, und eines wässrigen Stroms (21), der Melamin enthält und CO₂-verarmt ist;
e) Rückgewinnen des in dem wässrigen CO₂-verarmten Rohmelamin-Strom (4) enthaltenen Melamins und des in dem wässrigen Strom (21), der Melamin enthält und CO₂-verarmt ist, enthaltenen Melamins durch Kristallisation durch Abkühlung unter Bildung eines Stroms (10) von kristallisiertem Melamin und eines Mutterlaugenstroms (23).

2. Verfahren nach Anspruch 1, **dadurch gekennzeichnet, dass**, bevor der CO₂-verarmte Rohmelamin-Flüssigkeitsstrom (4) der Kristallisation durch Abkühlung unterzogen wird, er einer Quench-Ammonolysebehandlung durch Kontakt mit einem wässrigen Ammoniakstrom (36) unter Bildung eines wässrigen Ammoniakstroms (35), der gereinigtes Melamin enthält, unterzogen wird.

3. Verfahren nach Anspruch 2, **dadurch gekennzeichnet, dass** der wässrige Strom (21), der Melamin enthält und CO₂-verarmt ist, mit dem gereinigtes Melamin enthaltenden wässrigen Ammoniakstrom (35) unter Bildung eines einzigen Stroms (7), der Schritt e) unterzogen wird, verbunden wird.

4. Verfahren nach einem der vorstehenden Ansprüche, **dadurch gekennzeichnet, dass** in Schritt c) das Entfernen von CO₂ aus dem Melamin, NH₃ und CO₂ enthaltenden wässrigen Strom (20) mittels Druckabsenkung bewirkt wird.

5. Verfahren nach einem der Ansprüche 1 bis 3, **dadurch gekennzeichnet, dass** in Schritt d) das Entfernen von CO₂ aus dem Melamin, NH₃ und CO₂ enthaltenden wässrigen Strom (20) mittels Stripping bewirkt wird.

6. Verfahren nach einem der vorstehenden Ansprüche, **dadurch gekennzeichnet, dass** der Strom (22), der das in Schritt d) erhaltene entfernte CO₂ enthält, zu einem Schritt desselben Verfahrens auf Schritt e) folgend für die Rückgewinnung von Melamin durch Kristallisation durch Kühlung rückgeführt wird.

7. Verfahren nach einem der vorstehenden Ansprüche, **dadurch gekennzeichnet, dass** der feuchte Abgasstrom (19), der Melamin, NH₃, CO₂ und Wasserdampf enthält, zu einer Harnstofferzeugungsanlage rückgeführt wird, und zwar unverändert oder nachdem er einer Kondensationsbehandlung durch Absorption in wässriger Lösung oder einer anderen Art von Behandlung unterzogen wurde.

8. Verfahren nach Anspruch 2, **dadurch gekennzeichnet, dass** mindestens ein Teil des in Schritt e) erhaltenen Mutterlaugestroms (23) als wässriger Strom (25) bei der Quench-Ammonolysebehandlung des CO₂-verarmten Rohmelamin-Flüssigkeitsstroms (4) verwendet wird.

9. Verfahren nach einem der vorstehenden Ansprüche, **dadurch gekennzeichnet, dass** mindestens ein Teil des in Schritt e) erhaltenen Mutterlaugestroms (23) einer Deammonisierungsbehandlung unter Bildung eines im Wesentlichen CO₂-freien gasförmigen NH₃-Stroms (26), eines CO₂ enthaltenden Stroms (27) und eines wässrigen Stroms (28) aus deammonisierter Mutterlauge unterzogen wird.

10. Verfahren nach dem vorstehenden Anspruch, **dadurch gekennzeichnet, dass** der CO₂ enthaltende Strom (27) direkt oder nach einer Behandlung, die seinen Wassergehalt reduziert, zu einer Harnstofferzeugungsanlage rückgeführt wird.

11. Verfahren nach Anspruch 9 oder 10, **dadurch gekennzeichnet, dass** der wässrige Strom (28) aus deammonisierter Mutterlauge einer Zersetzungsbehandlung unter Rückgewinnung von NH₃ und CO₂ und der Erzeugung eines wässrigen Stroms unterzogen wird, der als wässriger Waschstrom (32) teilweise zu Schritt c) rückgeführt wird und teilweise als wässriger Strom (31) rückgeführt wird, um zur Bildung des wässrigen Ammoniakstroms (36) zu der Quench-Ammonolysebehandlung beizutragen.

12. Verfahren nach Anspruch 9 oder 10, **dadurch gekennzeichnet, dass** der wässrige Strom (28) aus deammonisierter Mutterlauge getrennt wird in:
i) einen Melamin-angereicherten wässrigen Strom, der teilweise als wässriger Waschstrom (32) zu Schritt b) rückgeführt wird und teilweise als wässriger Strom (31) rückgeführt wird, um zur Bildung des wässrigen Ammoniakstroms (36) zu der Quench-Ammonolysebehandlung beizutragen;
ii) einen OAT-angereicherten wässrigen Strom, der dafür geeignet ist, mit der Rückgewinnung von NH₃ und CO₂ ersetzt zu werden.

13. Verfahren nach den Ansprüchen 11 oder 12, **dadurch gekennzeichnet, dass** der im Wesentlichen CO₂-freie wässrige NH₃-Strom (26) mit einem Teil des wässrigen Stroms (31) gemischt wird, um die Kondensationswärme von diesem rückzugewinnen.

14. Verfahren nach einem der vorstehenden Ansprüche, **dadurch gekennzeichnet, dass** die Schritte a) und b) zur gleichen Zeit unter Bildung eines einzelnen wasserfreien Abgasstroms, der NH₃, CO₂ und Melamindampf enthält, und eines CO₂-verarmten Rohmelaminstroms bewirkt werden, wobei der einzelne Strom von wasserfreiem Abgas zu Schritt c) geleitet wird.

15. Vorrichtung zur Anwendung des verbesserten Verfahrens für die Herstellung von Melamin gemäß der Definition in Anspruch 1, enthaltend:
i) einen Trennungsabschnitt zum Trennen eines zweiphasigen Flüssigkeit-/Gas-Abflusses, der in einer Harnstoff-Pyrolysereaktion erzeugt wurde, in einen Rohmelamin-Flüssigkeitsstrom (3) und einen ersten wasserfreiem Abgasstrom (15), der NH₃, CO₂ und Melamindämpfe enthält, welcher Trennungsabschnitt mit einem Reaktionsabschnitt (R) für die Harnstoff-Pyrolyse verbunden ist, von welchem er den zweiphasigen Flüssigkeit-/Gas-Abfluss erhält, welcher Trennungsabschnitt innerhalb oder außerhalb des Reaktionsabschnitts (R) ist;
ii) einen Strippingabschnitt (StrN), um den Rohmelamin-Flüssigkeitsstrom, der von dem Reaktionsabschnitt (R) kommt, mit einem wasserfreien gasförmigen NH₃-Strom in Kontakt zu bringen und einen CO₂-verarmten Rohmelamin-Flüssigkeitsstrom (4) und einen zweiten wasserfreien Abgasstrom (16), der NH₃, CO₂ und Melamindampf enthält, zu bilden, welcher Strippingabschnitt (StrN) mit dem Reaktionsabschnitt (R) verbunden ist, von welchem er den Rohmelamin-Flüssigkeitsstrom (4) erhält;
iii) einen Waschabschnitt (OGQ), um den ersten und den zweiten wasserfreiem Abgasstrom (15, 16) mit mindestens einem wässrigen Waschstrom (32) in Kontakt zu bringen und einen wässrigen Strom (20), der Melamin, NH₃, und CO₂ enthält, und einen feuchten Abgasstrom (19), der NH₃, CO₂ und Wasserdampf enthält, zu bilden, welcher Waschabschnitt (OGQ) mit dem Trennungsabschnitt, von welchem er den ersten wasserfreien Abgasstrom (15) erhält, und mit dem Strippingabschnitt (StrN) verbunden ist, von welchem er den zweiten wasserfreien Abgasstrom (16) erhält;
iv) einen CO₂-Entfernungsabschnitt (OGS) zum Entfernen zumindest eines Teils des darin enthaltenen CO₂ aus dem Melamin, NH₃ und CO₂ enthaltenden wässrigen Strom (20) und Bilden eines Stroms (22), der das entfernte CO₂ enthält, und eines wässrigen Stroms (21), der Melamin enthält und CO₂-verarmt ist, welcher CO₂-Entfernungsabschnitt (OGS) mit dem Waschabschnitt (OGQ) verbunden ist, von welchem er den Melamin, NH₃ und CO₂ enthaltenden wässrigen Strom (20) erhält;
v) mindestens einen Melamin-Rückgewinnungsabschnitt (Cr), um sowohl das in dem CO₂-verarmten wässrigen Rohmelamin-Strom (4) enthaltene Melamin als auch das in dem Melamin und CO₂ enthaltenden wässrigen Strom (21) enthaltene Melamin durch Kühlungskristallisation unter Bildung eines Stroms (10) von kristallisiertem Melamin und eines Mutterlaugestroms (23) zu gewinnen, welcher Rückgewinnungsabschnitt (Cr) von Melamin sowohl mit dem Strippingabschnitt (StrN), von welchem er den CO₂-verarmten Rohmelamin-Flüssigkeitsstrom (4) erhält, als auch dem CO₂-Entfernungsabschnitt (OGS) verbunden ist, von welchem er den Melamin enthaltenden und CO₂-verarmten wässrigen Strom (21) erhält.

## Revendications

1. Processus de consommation à basse énergie pour la production de mélamine de pureté élevée, par la pyrolyse d'urée, comprenant les étapes opérationnelles suivantes :
a) séparation d'un produit biphasique d'effluent de liquide-gaz dans une réaction de pyrolyse d'urée en un flot liquide de mélamine brute (3) et en un premier flot de dégagement gazeux anhydre (15) comprenant du NH₃, du CO₂ et des vapeurs de mélamine ;
b) mise dudit flot liquide de mélamine brute (3) en contact avec un flot de NH₃ anhydre gazeux (13) et formation d'un flot liquide de mélamine brute appauvri en CO₂ (4) et d'un second flot de dégagement gazeux anhydre (16) comprenant du NH₃, du CO₂ et des vapeurs de mélamine ;
c) mise desdits premier et second flots de dégagement gazeux anhydres (15, 16) en contact avec au moins un flot de lavage aqueux (32) et formation d'un flot aqueux (20) comprenant de la mélamine, du NH₃, du CO₂ et un flot de dégagement gazeux humide (19) comprenant du NH₃, du CO₂ et de la vapeur d'eau ;
d) enlèvement depuis ledit flot aqueux (20) comprenant de la mélamine, du NH₃, du CO₂, d'au moins une partie du CO₂ contenu en son sein, et formation d'un flot (22) comprenant le CO₂ enlevé et d'un flot aqueux (21) comprenant de la mélamine et appauvri en CO₂ ;
e) récupération de la mélamine contenue dans ledit flot liquide (4) de mélamine brute appauvri en CO₂ et de la mélamine contenue dans ledit flot aqueux (21) comprenant de la mélamine et appauvri en CO₂ par l'intermédiaire d'une cristallisation par refroidissement, avec la formation d'un flot (10) de mélamine cristallisée et d'un flot (23) de liqueur mère.

2. Processus selon la revendication 1, **caractérisé en ce que**, avant que ledit flot liquide (4) de mélamine brute appauvri en CO₂ ne soit soumis à une cristallisation par refroidissement, il est soumis à un traitement de trempe-ammoniolyse par contact avec un flot d'ammoniac aqueux (36), avec la formation d'un flot d'ammoniac aqueux (35) comprenant de la mélamine purifiée.

3. Processus selon la revendication 2, **caractérisé en ce que** ledit flot aqueux (21) comprenant de la mélamine et appauvri en CO₂ est joint audit flot d'ammoniac aqueux (35) comprenant de la mélamine purifiée, avec la formation d'un flot unique (7) à soumettre à ladite étape e).

4. Processus selon l'une quelconque des revendications précédentes, **caractérisé en ce que** dans l'étape c) l'enlèvement de CO₂ depuis ledit flot aqueux (20) comprenant de la mélamine, du NH₃ et du CO₂ est effectué au moyen d'une dépressurisation.

5. Processus selon l'une quelconque des revendications 1 à 3, **caractérisé en ce que** dans ladite étape d) l'enlèvement de CO₂ depuis ledit flot aqueux (20) comprenant de la mélamine, du NH₃ et du CO₂ est effectué au moyen d'un démontage.

6. Processus selon l'une quelconque des revendications précédentes, **caractérisé en ce que** ledit flot (22) comprenant le CO₂ enlevé obtenu dans l'étape d) est recyclé vers une étape du même processus, à la suite de ladite étape e) pour la récupération de mélamine par une cristallisation par refroidissement.

7. Processus selon l'une quelconque des revendications précédentes, **caractérisé en ce que** ledit flot de dégagement gazeux humide (19) comprenant de la mélamine, du NH₃, du CO₂ et de la vapeur d'eau est recyclé vers une installation de production d'urée, comme tel ou après avoir été soumis à un traitement de condensation par absorption dans une solution aqueuse ou un autre type de traitement.

8. Processus selon la revendication 2, **caractérisé en ce qu'**au moins une partie dudit flot de liqueur mère (23) obtenu dans l'étape e) est utilisée en tant que flot aqueux (25) dans le traitement de trempe-ammoniolyse dudit flot liquide de mélamine brute appauvri en CO₂ (4).

9. Processus selon l'une quelconque des revendications précédentes, **caractérisé en ce qu'**au moins une partie dudit flot de liqueur mère (23) obtenu dans l'étape e) est soumise à un traitement de désammoniation, avec la formation d'un flot gazeux de NH₃, sensiblement exempt de CO₂ (26), d'un flot (27) comprenant du CO₂ et d'un flot aqueux (28) de liqueur mère désammoniée.

10. Processus selon la revendication précédente, **caractérisé en ce que** ledit flot (27) comprenant du CO₂ est recyclé vers une installation de production d'urée, directement ou après un traitement qui réduit sa teneur en eau.

11. Processus selon la revendication 9 ou 10, **caractérisé en ce que** ledit flot aqueux (28) de liqueur mère désammoniée est soumis à un traitement de décomposition avec la récupération de NH₃ et de CO₂, et la production d'un flot aqueux qui est partiellement recyclé, en tant que flot de lavage aqueux (32), vers ladite étape c) et partiellement recyclé en tant que flot aqueux (31) pour contribuer à la formation dudit flot d'ammoniac aqueux (36) vers ledit traitement de trempe-ammoniolyse.

12. Processus selon la revendication 9 ou 10, **caractérisé en ce que** ledit flot aqueux (28) de liqueur mère désammoniée est séparé en :
i) un flot aqueux enrichi en mélamine qui est partiellement recyclé en tant que flot de lavage aqueux (32) à ladite étape b) et partiellement recyclé en tant que flot aqueux (31) pour contribuer à la formation dudit flot d'ammoniac aqueux (36) vers ledit traitement de trempe-ammoniolyse ;
ii) un flot aqueux enrichi en OAT, approprié pour être décomposé avec la récupération de NH₃ et de CO₂.

13. Processus selon les revendications 11 ou 12, **caractérisé en ce que** ledit flot aqueux (26) de NH₃ sensiblement exempt de CO₂ est mélangé avec une partie dudit flot aqueux (31) pour récupérer la chaleur de condensation à partir de là.

14. Processus selon l'une quelconque des revendications précédentes, **caractérisé en ce que** lesdites étapes a) et b) sont effectuées en même temps avec la formation d'un flot unique de dégagement gazeux anhydre comprenant du NH₃, du CO₂ et de la vapeur de mélamine et d'un flot de mélamine brute appauvri en CO₂, ledit flot unique de dégagement gazeux anhydre étant envoyé à l'étape c).

15. Équipement pour appliquer le processus amélioré pour la production de mélamine selon la revendication 1, comprenant :
i) une section de séparation pour séparer un effluent biphasique liquide / gaz produit dans une réaction de pyrolyse d'urée en un flot liquide de mélamine brute (3) et en un premier flot de dégagement gazeux anhydre (15) comprenant du NH₃, du CO₂ et de la vapeur de mélamine, ladite section de séparation étant reliée à une section de réaction (R) pour la pyrolyse d'urée à partir de laquelle elle reçoit ledit effluent biphasique liquide / gaz, ladite section de séparation étant à l'intérieur ou à l'extérieur de ladite section de réaction (R) ;
ii) une section de démontage (StrN) pour mettre ledit flot liquide de mélamine brute provenant de ladite section de réaction (R) en contact avec un flot gazeux anhydre de NH₃ formant un flot liquide de mélamine brute appauvri en CO₂ (4) et un second flot de dégagement gazeux anhydre (16) comprenant du NH₃, du CO₂ et de la vapeur de mélamine, ladite section de démontage (StrN) étant reliée à ladite section de réaction (R) à partir de laquelle elle reçoit ledit flot liquide de mélamine brute (4) ;
iii) une section de lavage (OGQ) pour mettre lesdits premier et second flots de dégagement gazeux anhydre (15, 16) en contact avec au moins un flot de lavage aqueux (32) et former un flot aqueux (20) comprenant de la mélamine, du NH₃ et du CO₂ et un flot de dégagement gazeux humide (19) comprenant du NH₃, du CO₂ et de la vapeur d'eau, ladite section de lavage (OGQ) étant reliée à ladite section de séparation à partir de laquelle elle reçoit ledit premier flot de dégagement gazeux anhydre (15) et à ladite section de démontage (StrN) à partir de laquelle elle reçoit ledit second flot de dégagement gazeux anhydre (16) ;
iv) une section d'enlèvement de CO₂ (OGS) pour l'enlèvement, à partir dudit flot aqueux (20) comprenant de la mélamine, du NH₃ et du CO₂, d'au moins une partie du CO₂ contenu en son sein, et la formation d'un flot (22) comprenant le CO₂ enlevé et d'un flot aqueux (21) comprenant de la mélamine et appauvri en CO₂, ladite section d'enlèvement de CO₂ (OGS) étant reliée à ladite section de lavage (OGQ) à partir de laquelle elle reçoit ledit flot aqueux (20) comprenant de la mélamine, du NH₃ et du CO₂ ;
v) au moins une section de récupération de mélamine (Cr) pour récupérer tant la mélamine contenue dans ledit flot liquide de mélamine brute appauvri en CO₂ (4) que la mélamine contenue dans ledit flot aqueux (21) comprenant de la mélamine et du CO₂ par une cristallisation par refroidissement, avec la formation d'un flot (10) de mélamine cristallisée et d'un flot (23) de liqueur mère, ladite section de récupération (Cr) de la mélamine étant reliée tant à la section de démontage (StrN) à partir de laquelle elle reçoit ledit flot liquide (4) de mélamine brute appauvri en CO₂ qu'à ladite section d'enlèvement de CO₂ (OGS) à partir de laquelle elle reçoit ledit flot aqueux (21) comprenant de la mélamine et appauvri en CO₂.
